(19) Europäisches Patentamt
European Patent Office
Office européen des brevets

(11) Veröffentlichungsnummer: **0 306 699 A1**

# (12) EUROPÄISCHE PATENTANMELDUNG

(21) Anmeldenummer: **88112494.5**

(22) Anmeldetag: **01.08.88**

(51) Int. Cl.4: **A61K 31/44 , A61K 9/24 , A61K 9/54**

Die Bezeichnung der Erfindung wurde geändert (Richtlinien für die Prüfung im EPA, A-III, 7.3).

(30) Priorität: **11.08.87 DE 3726666**
**26.03.88 DE 3810350**

(43) Veröffentlichungstag der Anmeldung: **15.03.89 Patentblatt 89/11**

(84) Benannte Vertragsstaaten: **AT BE CH DE ES FR GB GR IT LI LU NL SE**

(71) Anmelder: **BAYER AG**
**Konzernverwaltung RP Patentabteilung**
**D-5090 Leverkusen 1 Bayerwerk(DE)**

(72) Erfinder: **Ohm, Andreas, Dr.**
**Am Röttgen 42**
**D-4040 Neuss(DE)**
Erfinder: **Luchtenberg, Helmut, Dr.**
**Windmuehlenstrasse 11a**
**D-5216 Niederkassel 6(DE)**
Erfinder: **Bücheler, Manfred**
**Lorkenhoehe 49**
**D-5063 Overath 2(DE)**
Erfinder: **Schmoll, Josef**
**Irlenweg 6**
**D-5632 Wermelskirchen 2(DE)**
Erfinder: **Rupp, Roland, Dr.**
**Solinger Strasse 18**
**D-5653 Leichlingen 2(DE)**
Erfinder: **Porges, Eduard, Dr.**
**Ahornstrasse 13**
**D-5000 Koeln 80(DE)**
Erfinder: **Nishioka, Takaaki**
**1072-15, Oharanaka Koka-cho**
**Koka-gun Shiga-Prefecture(JP)**

## (54) Dihydropyridin-Retard-Zubereitung.

(57) Die Erfindung betrifft feste Arzneizubereitungen mit lang anhaltender Wirkung enthaltend einen schwer löslichen Dihydropyridinwirkstoff der allgemeinen Formel I

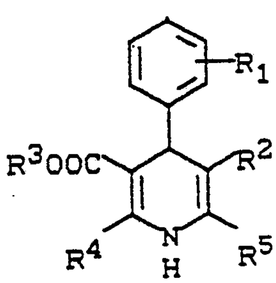

I,

in welcher $R^1$ bis $R^5$ die in der Beschreibung angegebene Bedeutung haben, sowie Verfahren zu ihrer Herstellung, bestehend aus einem Kern sowie einem darum angebrachten Mantel.

EP 0 306 699 A1

DHP-Retard-Zubereitung

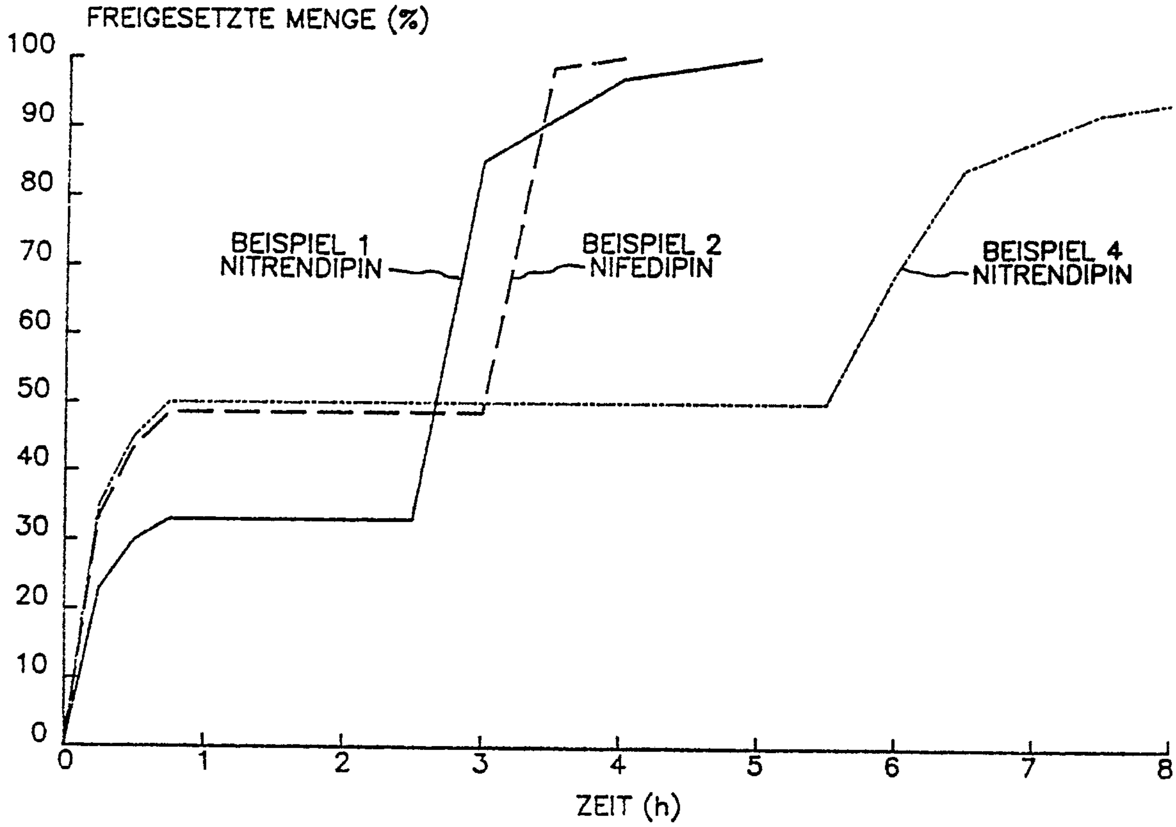

## DHP-Retard-Zubereitung

Die Erfindung betrifft feste Arzneizubereitungen mit lang anhaltender Wirkung für Dihydropyridine bestehend aus einem schnell freisetzenden Kern mit einem darum befindlichen den Freisetzungsbeginn verzögernden Mantel sowie Verfahren zu ihrer Herstellung.

Wirkstoffe aus der Stoffklasse der Dihydropyridine und ihre Verwendung als Kreislaufmittel sind bereits bekannt (vgl. Brit. Pat. 1 173 862, Brit. Pat. 1 358 951, US-Pat. 4 256 749, DE-OS 3 311 003 und US-Pat. 4 264 611). Bei der galenischen Zubereitung dieser potenten Wirkstoffe treten häufig Schwierigkeiten auf, da die Stoffe nur eine sehr geringe Löslichkeit besitzen, häufig lichtempfindlich sind und ihre Resorbierbarkeit in biologischen Systemen häufig zu Problemen führt.

Einige Dihydropyridine weisen zudem eine besondere Pharmakokinetik auf: ihr Metabolismus erfolgt vorwiegend bei der ersten Leberpassage (sog. first pass effect), die direkt nach der Resorption des Wirkstoffes erfolgt. Dabei hängt bei diesen Wirkstoffen die Metabolisierungsrate von der Anflutungsgeschwindigkeit des Wirkstoffes in die Leber ab: mit zunehmender Menge pro Zeiteinheit wird prozentual weniger Wirkstoff abgebaut (sog. "sättigbarer first pass Effekt"). Die Bioverfügbarkeit des Wirkstoffes ist dann abhängig von der in vivo Freisetzungsrate des Wirkstoffes.

Es wurden zahlreiche Versuche unternommen, optimale galenische Zubereitungen herzustellen, die die Bioverfügbarkeit dieser potenten Wirkstoffe verbessern. So wurden z.B. einige Wirkstoffe in speziellen organischen Lösungsmittelsystemen gelöst und in Gelatinekapseln eingefüllt, um einen schnellen und effektiven Wirkungseintritt zu gewährleisten (vgl. Brit. Pat. 1 362 627). Es wurde auch versucht, Dihydropyridine wie Nifedipin unter Verwendung von wasserlöslichen Polymeren in Copräzipitate bzw. "feste Lösungen" zu überführen, um die Bioverfügbarkeit zu verbessern (vgl. Brit. Pat. 1 579 818). Des weiteren wurden z. B. Nifedipin-Zubereitungen mit verlängerter Wirkungsdauer beschrieben, die kristallines Nifedipin mit einer ausgewählten spezifischen Oberfläche enthalten (vergl. EP-A-0 047 899). Diese galenischen Zubereitungsformen besitzen jedoch den Nachteil, daß nach einigen Stunden die Plasmaspiegel absinken und damit Abschwächungen der Wirkung eintreten können. Demgegenüber zeigen die erfindungsgemäßen Zubereitungen gegen Ende der Resorptionszeit einen Wirkstoffsto der zu einer höheren Wirksamkeitsrate führt, woduch auch bei einmal täglichen Applikationen eine sichere Wirkung erzielt wird.

Für die Behandlung von Krankheiten, die über längere Zeiträume behandelt werden müssen, wie z. B. Hypertonie ist es wünschenswert, die Häufigkeit der Einnahme von Medikamenten so gering wie möglich zu halten. Dies ist nicht nur angenehmer für den Patienten, sondern es erhöht auch die Behandlungssicherheit, indem es die Nachteile unregelmäßiger Einnahmen vermindert und zu einem gleichmäßigeren Wirkstoffkonzentrationsverlauf im Körper führt. Dadurch wird gleichzeitig das Risiko von unerwünschten Über- bzw. Unterdosierungen minimiert. Durch den Einsatz der erfindungsgemäßen Zubereitungen werden Blutspiegelspitzen nach Einnahme von schnell freisetzenden Formen vermieden und das Risiko durch unregelmäßige oder vergessene Einnahme bei häufiger Applikationsfrequenz vermindert.

Von besonderem Vorteil sind Darreichungsformen, die den Wirkstoff entsprechend dem Bedarf des Patienten abgeben. So ist z. B. für den Verlauf des Blutdrucks ein Tagesrhythmus beschrieben, (vergl lemmer B.; Chronopharmakologie, Tagesrhythmen und Arzneimittelwirkung, Wiss. Verlag GmbH Stuttgart (1984) ) wonach während der Nacht sowohl die normotonen als auch die hypertonen Werte (systolisch) auf ein Minimum (gegen 4 Uhr morgens) absinken und darauf hin in den frühen Morgenstunden, d. h. bereits während des Schlafes, steil wieder anzusteigen. Der Blutdruck erreicht dann ein Maximum gegen 10 Uhr vormittags. Ein sich dem Tagesrhythmus anpassendes Blutdruckmedikament hätte gegenüber bisherigen retardierten Systemen den Vorteil, dem Patienten nur dann mit Wirkstoff zu belasten, wenn dieser vom Körper benötigt wird. Insbesondere kann der Blutdruck, der bereits in den frühen Morgenstunden während des Schlafes steil ansteigt, durch die Abgabe größerer Wirkstoffmengen aus dem Kern sicher abgefangen werden, ohne daß der Körper in den vorausgehenden Nachtstunden mit ihrem normalen Blutdruckabfall mit nicht genötigtem Wirkstoff belastet wird. Bei einer abendlichen Einnahme der erfindungsgemäßen Zubereitung kann somit eine optimale Blutplasmakonzentration, die an den biologischen Tagesrhythmus des Patienten angepaßt ist, gewährleistet werden.

Sowohl für den Arzt als auch für den Patienten besteht ein Bedürfnis, z. B. für die Dauertherapie von Kreislauferkrankungen, die hochwirksamen Dihydropyridine in einer Form zur Verfügung gestellt zu bekommen, daß möglichst eine einmal tägliche Applikation zur Krankheitsbehandlung ausreicht.

Für Dihydropyridine wurden bereits Arzneizubereitungen mit verzögerter Wirkstoff-Freigabe (Retard-Formen) beschrieben. So wurde z.B versucht, durch eine spezielle Korngrößenverteilung des kristallinen Wirkstoffes bzw. durch eine ausgewählte spezifische Oberfläche der Wirkstoffkristalle eine langsam freisetzende Zubereitung herzustellen (vgl. DE-OS 3 033 919). Weiterhin wurden spezielle Tablettenzubereitungen

vorgeschlagen, die nach dem Prinzip der osmotischen Pumpe den Wirkstoff aus dem Inneren einer Tablette, die mit einer semipermeablen Lackschicht umgeben ist, durch eine vorgegebene Öffnung über einen längeren Zeitraum freisetzen und somit einen Retard-Effekt erzielen (vgl. US-PS 3 916 899).

Die bisher bekannten Zubereitungsformen mit verzögerter Wirkstoffabgabe, insbesondere solche für Dihydropyridine, weisen eine Reihe von Nachteilen auf. Ihre Retard-Wirkung ist entweder nur auf einige Stunden beschränkt, so daß der Patient in der Regel nach wie vor zwei- oder mehrmals täglich applizieren muß oder die Freisetzungsgeschwindigkeit des Wirkstoffes läßt nach einigen Stunden deutlich nach, so daß auch die Blutspiegel unter die erforderliche Effektivitätsgrenze absinken können.

Bei dem oben erwähnten osmotischen System kann es im Magen oder Darmtrakt je nach eingesetzter Kapselfüllung zu lokalen Irritationen des Gewebes durch überhöhte Konzentrationen kommen. Weiterhin ist auch bei diesem osmotischen Retardierungsprinzip eine Abflachung der Freisetzungskurve im terminalen Bereich zu beobachten. Bedingt durch die Natur des osmotischen Systems kann ein Teil des Wirkstoffs in der Arzneiform verbleiben und somit nicht für die gewünschte Resorption zur Verfügung stehen. Die Herstellung dieser Arzneiform ist zudem sehr aufwendig, da hierbei organische Lösungsmittel im Herstellungsprozeß eingesetzt werden müssen und die Lackschicht jeder Tablette einzeln mit Hilfe spezieller Techniken z. B. mit einem Laserstrahl durchbohrt werden muß.

Im Fall der oben erwähnten Dihydropyridine, die eine von der in vivo-Freisetzungsrate abhängige Bioverfügbarkeit aufweisen, führen die bisher erwähnten kontinuierlich freisetzenden Retardformulierungen zudem zu vergleichsweise niedrigen Bioverfügbarkeiten.

Aus dem Stand der Technik ist ebenfalls bekannt, Arzneizubereitungen mit einer magensaftresistenten Lackierung zu überziehen. Bei diesen Arzneimitteln wird ein Teil oder die gesamte Dosis des Wirkstoffs erst nach dem Verlassen des Magens aus der Arzneiform freigesetzt. Diese diskontinuierlich freisetzenden Retardformen sind allein schon aufgrund ihrer pH-Abhängigkeit der Wirkstoffliberation unzuverlässig, da die Magenverweilzeiten von Patent zu Patient extrem streuen und zudem wesentlich von der Nahrungsmittelaufnahme abhängen (Magenverweilzeiten ca. 0,2 - 12 h). Mit einer solchen Arzneiform kann zwar ein Teil des Wirkstoffs zeitlich versetzt freigesetzt werden, die Reproduzierbarkeit dieser Verzögerungsphase (lag time) ist allerdings aus den obengenannten Gründen nicht gegeben, so daß z. B. eine sichere Anpassung an Tagesrhythmus abhängige biologische Prozesse hiermit nicht erreicht werden kann.

Es wurde nun gefunden, daß feste Arzneizubereitungen mit lang anhaltender Wirkung für 1 bis maximal 2 mal tägliche Applikation, bestehend aus einem schnell freisetzenden Kern und einem den Freisetzungsbeginn verzögernden Mantel für Dihydropyridinwirkstoffe besonders geeignet sind und überraschenderweise eine hohe Effektivität und einen sicheren Zeitablauf der Resorption gewährleisten.

Gegenstand der Erfindung sind feste Arzneizubereitungen mit lang anhaltender Wirkung für 1 bis maximal 2 mal tägliche Applikation bestehend aus einem schnell freisetzenden Kern und einem den Freisetzungbeginn verzögernden Mantel enthaltend mindestens ein Dihydropyridin der allgemeinen Formel I

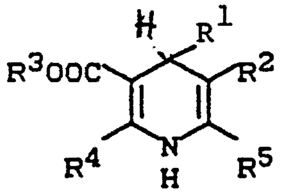

I,

in welcher

$R^1$ für einen Phenylrest steht, der ein oder zwei gleiche oder verschiedene Substituenten aus der Gruppe Nitro, Halogen oder Trifluormethyl trägt oder für den Rest

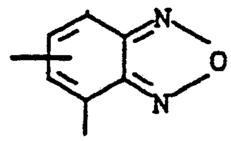 oder

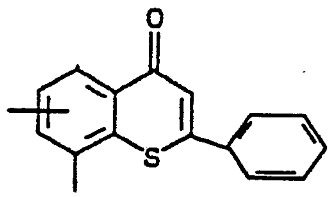 steht,

$R^2$ für eine Nitrogruppe steht oder für den Rest $COOR_6$ steht, wobei
$R_6$ Alkyl mit 1 bis 10 C-Atomen bedeutet, welches gegebenenfalls substituiert ist durch Alkoxy mit 1 bis 4 C-Atomen oder durch ein oder mehrere Halogene
oder wobei
$R^2$ gemeinsam mit $R^5$ für die Lactongruppe $-CO-O-CH_2-$ steht,
$R^3$ für Alkyl mit 1 bis 10 C-Atomen steht, welches gegebenenfalls substituiert ist durch Alkoxy mit 1 bis 4 C-Atomen oder durch ein oder mehrere Fluore und
$R^4$ und $R^5$ gleich oder verschieden sind und jeweils für Alkyl mit 1 bis 4 C-Atomen, welches gegebenenfalls durch Hydroxy substituiert ist, stehen,
wobei die Kern-Mantel-Darreichungsform

a) aus einem Kern besteht, der mindestens eines der obengenannten Dihydropyridine in schnell freisetzender Form enthält und

b) aus einem um den Kern liegenden Mantel besteht, der keinen Wirkstoff enthält und der sich in wäßrigem Milieu nur langsam auflöst, und

c) gegebenenfalls auf dem Mantel zusätzlich eine schnell freisetzende Initialdosis des Dihydropyridinwirkstoffs enthält, wobei der Durchmesser der Zubereitungsform zwischen 0,5 und 15 mm liegt.

Bevorzugt sind solche Zubereitungen, die im Kern 20 - 100 Gewichtsprozent, vorzugsweise 40 - 100 Gewichtsprozent, insbesondere 50 - 100 Gewichtsprozent des gesamten Dihydropyridinwirkstoffs der Darreichungsform enthalten.

Falls eine schnell freisetzende Initialdosis auf den Mantel aufgebracht wird, enthält diese vorzugsweise 10 - 80 Gewichtsprozent, insbesondere 15 - 50 Gewichtsprozent der gesamten Dihydropyridinmenge.

Je nach Art des Wirkstoffs enthalten die erfindungsgemäßen Zubereitungen insgesamt vorzugsweise 1 bis 200 mg, insbesondere 10 bis 150 mg mindestens eines Wirkstoffs aus der Klasse der Dihydropyridine.

Der schnell freisetzende Kern der Zubereitung enthält den Wirkstoff vorzugsweise in amorpher Form oder in fein gemahlener bzw. mikronisierter kristalliner Form.

Als Kern mit schneller Freisetzung werden vorzugsweise solche Kerne verstanden, die den Dihydropyridinwirkstoff in nicht retardierter Form enthalten und ihn zu mindestens 75 % in einer Zeit von maximal 1 Stunde freisetzen unter den folgenden Freisetzungsbedingungen:

USP-Paddle Methode unter Anwendung von "Sink Bedingungen", d. h. solchen Bedingungen, bei denen das wäßrige Freisetzungsmedium so gewählt wird in Art und Menge, daß es mindestens die dreifache Menge des eingesetzten Wirkstoffs lösen kann.

Der Ausdruck "Sink Bedingungen" sei näher erläutert wie folgt:
4 l 0,1 N Salzsäure + 0,1 bis 0,5 % Netzmittel wie Tween 80 oder Texapon K12 (Natriumlaurylsulfat); 37° C; 100 Upm.

Von besonderem Interesse sind die bekannten Dihydropyridine Nifedipin, Nitrendipin, Nisoldipin und Nimodipin.

Falls der schnell freisetzende Kern amorphes Dihydropyridin enthält, wird dieses vorzugsweise gemeinsam mit wasserlöslichen Polymeren wie Polyvinylpyrrolidon, Methylcellulose, Hydroxy propylcellulose oder Hydroxypropylmethylcellulose in organischem Lösungsmittel wie Aceton, Methylenchlorid, Chloroform oder niedrigen aliphatischen Alkoholen gelöst. Hierbei ist es zweckmäßig, auf 1 Gew.-Teil Dihydropyridin 2 bis 10 Gew.-Teile, insbesondere 3 bis 8 Gew.-Teile der wasserlöslichen Polymere einzusetzen und hieraus entsprechende Copräzipitate herzustellen.

Falls der schnell freisetzende Kern Dihydropyridine in kristalliner Form enthält, werden vorzugsweise Dihydropyridinkristalle mit einer maximalen mittleren Korngröße von 25 µm, insbesondere einer maximalen mittleren Korngröße von 15 µm eingesetzt. Die Bestimmung der Korngröße erfolgt nach der Cilas-Methode

(Lit.: A. Buerkholz et al, Part. Charact. 1, 1984, 153-160, "Laser diffraction spectrometers/experience in particle size analysis".).

Bei Verwendung von kristallinem Dihydropyridin im Kern ist die Zugabe von leicht wasserlöslichen Hilfsstoffen wie z.B. Lactose zweckmäßig. Ebenfalls kann durch den Einsatz von Sprengmitteln, wie z.B. quervernetztem Polyvinylpyrrolidon (PVP) oder durch oberflächenaktive Substanzen, wie z.B. Natriumlaurylsulfat, die Freisetzungsrate beschleunigt werden.

Der Mantel enthält keinen Wirkstoff. Im Gemisch mit pharmazeutisch üblichen Hilfsstoffen wie z.B. Laktose, Stärke, Cellulose, Citronensäure u.a. und Magnesiumstearat als Schmiermittel bildet ein hydrophiles gelbildendes Polymer das Mantelmaterial. Diese hydrophile Polymer steuert die Auflösungsgeschwindigkeit und Erosion des Mantels. Steuerungsfaktoren für die Ablösungs/Erosionsgeschwindigkeit des Mantelmaterials sind u.a. die Schichtdicke des Mantels und das Verhältnis Polymer(e)/restliche Hilfsstoffe.

Als hydrophile gelbildende Polymere sind z. B. modifizierte Stärken und celluloseartige Polymere wie z. B. Methylcellulose, Hydroxypropylmethylcellulose, Hydroxypropylcellulose und Na triumcarboxymethylcellulose geeignet, sowie gegebenenfalls als Modulator hydrophobe Hilfsstoffe wie hydriertes Rizinusöl, Calcium- und Magnesiumstearat, Stearinsäure, Cetylpalmitat und Wachse wie z. B. Bienenwachs.

Die Erosions- und Auflösungsgeschwindigkeit des Mantels kann auch gesteuert werden über die unterschiedlichen Viskositätsgrade des Polymeren, wobei die Geschwindigkeit sich erhöht, wenn niederviskose Qualitäten eingesetzt werden und langsamer wird beim Einsatz hochviskoser Typen. Üblicherwise liegt die Konzentration des Polymeren im Mantelmaterial bei 5 - 100 %, vorzugsweise bei 10 - 90 %. Die eingesetzte Konzentration ist abhängig vom Viskositätsgrad des(r) Polymeren und von der Löslichkeit/Hydrophilie der eingesetzten Hilfsstoffe und deren Menge.

Geeignete feste Darreichungsformen für das erfindungsgemäße Kern-Mantel-Prinzip sind z. B. Pellets und Manteltabletten. Die Größe der Zubereitung kann von 0,5 bis 15 mm variieren, wobei der untere Bereich bevorzugt für Pellets und der Bereich über 5 mm bevorzugt für Tabletten in Frage kommt.

Ein besonderer Vorteil bei der Verwendung von Pellets liegt in der Teilung der Gesamtwirkstoffdosis in mehrere Untereinheiten (sog. multiple dose), die es erlaubt, Pellets mit verschiedenen dicken oder mit verschieden zusammengesetzten Überzügen so zu kombinieren, daß das gewünschte Freisetzungsprofil der die Gesamtdosis enthaltenden Darreichungsformen (z. B. Steckkapsel) eingestellt werden kann, z. B. eine lineare Wirkstoffabgabe, eine kontinuierlich schneller werdende Freisetzung des Wirkstoffes oder eine pulsierende Abgabe des Wirkstoffes in zeitlich versetzten Stößen. Die Initialdosis kann hier z. B. durch nicht ummantelte Wirkstoffkerne eingebracht werden. Außerde kann durch das Kombinieren von Pelletkollektiven mit unterschiedlichen lag-Phasen eine Dosisminimierung erfolgen, dergestalt, daß die Blutplasmakonzentrationen den therapeutischen Bereich nicht verlassen und das Risiko von Nebenwirkungen vermindert wird.

Im Fall von Manteltabletten kann eine Initialdosis auf den Placebomantel aufgebracht werden, so daß hier eine diskontinuierliche, stoßweise Freisetzung erzielt wird.

Über die Mantelschichtdicke und die Anteile der Retardierungmittel lassen sich die gewünschten Verzögerungszeiten für die Wirkstofffreigabe aus dem Kern steuern. Die eingestellte Verzögerungszeit bis zur Freisetzung des Wirkstoffes im Kern durch Aberosion des Mantelmaterials bedingt den gewünschten Retardeffekt. Die Verzögerungszeit durch Aberosion/Ablösen des Mantels wird dabei praktisch nicht vom pH beeinflußt.

Es sei ausdrücklich darauf hingewiesen, daß die erfindungsgemäße Retard-Zubereitung sich von den bisher bekannten Kern-Mantel-Zubereitungen dadurch unterscheidet, daß der Mantel keinen Wirkstoff enthält und der Kern den Wirkstoff in schnell freisetzender Form enthält.

Die Herstellung der erfindungsgemäßen Manteltablette erfolgt nach üblichen Methoden, die durch folgendes Verfahren beispielhaft erläutert werden:

Zunächst wird der wirkstoffhaltige schnell freisetzende Kern mittels üblicher Tablettierprozesse hergestellt, z. B. aus Pulvermischungen oder Granulaten und anschließend wird um diesen Kern mit Hilfe einer Manteltablettenpresse (z. B. Kilian Presscoater) ein wirkstofffreier Placebomantel gelegt, wobei das Mantelmaterial aus einer rieselfähigen Pulvermischung oder aus einem Granulat, welches durch Trocken- oder Feuchtgranulation erhalten wurde, besteht und wobei gegebenenfalls auf die so erhaltenen Manteltabletten noch eine Lackschicht mit Hilfe üblicher Lackiermethoden, z. B. durch Aufsprühen oder durch Aufpressen eine weitere Schicht aufgebracht wird, die gegebenenfalls noch eine Initialdosis des Wirkstoffs enthält.

Die Herstellung von erfindungsgemäßen Mantel-Pellets erfolgt ebenfalls nach üblichen Methoden z. B. Granulatoren oder Pelletisiergeräten, die eine aufbauende Granulation ermöglichen z. B. in einem Pelletisierteller, einem Rotorgranulator, einem Wirbelschichtgranulator oder einem Dragierkessel, wobei die Pelletkerne entweder vorher gesondert hergestellt oder direkt kontinuierlich aufgebaut werden und anschließend das Mantelmaterial aufgebracht wird.

Besonders vorteilhaft ist die Verwendung von Rotorgranulatoren.

Übliche bekannte galenische Maßnahmen wie z. B. das Lackieren des Kerns, die Verwendung von Geschmacks- und Aromastoffen und Schmiermitteln und üblichen Hilfsmitteln, die dem galenischen Fachmann geläufig sind, können selbstverständlich auch bei der erfindungsgemäßen Zubereitung eingesetzt werden.

Aus dem Stand der Technik sind bereits Mehrschicht-Tabletten auf Basis von Casein-Matrices beschrieben, die zwei oder drei Schichten enthalten, die jeweils ihrerseits Wirkstoffe enthalten können (vgl. US-Pat. 3 184 386). Die dort beschriebenen Tabletten enthalten im Gegensatz zur vorliegenden Erfindung im äußeren Mantel Wirkstoff.

Auch in der US-Pat. 3 558 768 werden Manteltabletten beschrieben, die sowohl im Kern als auch im Mantel Wirkstoffe in langsam freisetzender Form enthalten. Die Freisetzungsgeschwindigkeiten können gemäß dieser US-Patentschrift verschieden sein, wobei es sich aber auf jeden Fall um langsam freisetzende Formen handelt.

Manteltabletten, die im Mantelmaterial keinen Wirkstoff enthalten, sind auch in Il Farmaco, No. 3, März 84, 67 f (Conte et al.) beschrieben. Die Freisetzungskinetik dieser Tabletten unterscheidet sich allerdings deutlich von dem hier beschriebenen erfindungsgemäßen Kern-Mantelprinzip: nach einer Verzögerungszeit wird der Wirkstoff über lange Zeit nach einer Kinetik nullter Ordnung kontinuierlich freigesetzt. Das Mantelmaterial dient hier als Diffusionsbarriere, nicht aber zur Einstellung einer diskontinuierlich erfolgenden stoßweisen Freisetzung des Wirkstoffes. Im Gegensatz zum vorliegenden erfindungsgemäßen Kern-Mantel-Prinzip kann die "Reservoirmanteltablette" nur bei Wirkstoffen mit einer gewissen Mindestwasserlöslichkeit eingesetzt werden.

Auch Salomon et al. (Pharm. Ind. 41. Nr. 8, S 799 f. 1979) beschreiben Manteltabletten, die keinen Wirkstoff im Mantelmaterial enthalten. Auch hier erfolgt die Freisetzung kontinuierlich (nach einer Wurzel-Zeit-Kinetik). Im Prinzip gilt das oben Gesagte. Eine weitere Darreichungsform, die speziell den sättigbaren first pass-Effekt von Psoralenen günstig beeinflußt, ist in DE 3 115 033 A 1 beschrieben. Das Mantelmaterial enthält hierbei allerdings Wirkstoff. Zudem wird die Verzögerung bis zur Freisetzung von Wirkstoff aus dem Kern der Manteltabletten bzw. Pellets nicht durch den Auftrag hoher Anteile an hydrophilen, gelbildenden Polymeren erreicht (Mantel), sondern durch eine Lackierung des Kerns/Pellets (ein dünner Lackfilm).

In der DE-OS 2 651 176 werden Pellets mit kontrollierter Wirkstoffabgabe beschrieben. Die dort genannten Formulierungen unterscheiden sich von den erfindungsgemäßen Mantelzubereitungen schon dadurch, daß sie auch im Mantel Wirkstoff enthalten. Zudem können die dort beschriebenen Formulierungen nur in aufwendigen Verfahren durch Aufbringen vieler Schichten erhalten werden, während die erfindungsgemäße Manteltablette durch einfaches Verpressen hergestellt wird und im Fall der Mantelpellets nur eine Schicht durch ein kontinuierliche arbeitendes Verfahren auf die schnell freisetzenden Kerne aufgebracht wird.

Durch das Prinzip der erfindungsgemäßen Zubereitung werden die üblichen Nachteile von bekannten Retard-Formen und auch von bisher bekannten Mehrschicht- oder Manteltabletten und Pellets bzw. von Zubereitungsformen die auf dem osmotischen Prinzip beruhen, vermieden. Insbesondere kann z. B. vermieden werden, daß die Freisetzungsrate des Wirkstoffs aufgrund des angestrebten Retardcharakters der Formulierung niedrig ist und damit z. B. niedrigere Bioverfügbarkeiten von Wirkstoffen wegen eines sättigbaren first pass-Effektes hingenommen werden müssen. Vielmehr wird der gesamte Wirkstoff bei Einsatz von Manteltabletten oder Mantelpellets mit einer einheitlichen Verzögerungszeit relativ schnell freigesetzt, aber in diskontinuierlichen Schüben, um den gewünschten Retardeffekt zu erzielen.

Durch diese gestaffelte, in vorbestimmbaren zeitlichen Abständen erfolgende Freisetzung, die zudem nicht pH-abhängig ist, unterscheidet sich die erfindungsgemäße Formulierung von allen bisher bekannten Retardierungsprinzipien für feste Darreichungsformen. Es wird erwartet, daß diese Formulierung besonders im Fall von Wirkstoffen, die eine sättigbare first pass-Kinetik besitzen, zu einer erhöhten Bioverfügbarkeit führt.

Ein weiterer Vorteil der erfindungsgemäßen Arzneiformen ist, daß sie besonders für solche Wirkstoffe geeignet sind, die in tieferen Abschnitten des Magen- Darmtraktes z. B. im Dickdarm eine höhere Resorption zeigen als im Magen oder im Dünndarm. Durch entsprechende Ausgestaltung des Mantels kann die lag Phase so bestimmt werden, daß der Wirkstoff im effektiv resorbierenden Teil des Magen-Darmtraktes freigesetzt wird, womit eine hohe Bioverfügbarkeit und ein gesicherter Effekt erzielt werden kann.

Die erfindungsgemäße Technologie ermöglicht zudem auch eine Anpassung der Wirkstofffreisetzung an den Blutdrucktagesrhythmus.

Als ein weiterer Vorteil ist die einfache Herstellungstechnologie zu nennen.

Im Fall der Mantelpellets (Arzneiform z. B. Kapsel) ist als weiterer zusätzlicher Vorteil die Möglichkeit der Einstellung beliebiger Freisetzungskinetiken des Wirkstoffes zu nennen (durch Kombination verschiedener Mantelpellets). Dadurch kann eine solche Retardzubereitung entsprechend den Anforderungen eines vorgegebenen Wirkstoffes quasi "maßgeschneidert" werden.

Im Hinblick auf das seit langem bestehende Bedürfnis nach Arzneizubereitungsformen mit lang anhaltender Wirkung ist es mehr als überraschend, daß bisher niemand die einfach herstellbare und sehr effektvolle erfindungsgemäße Mantelarzneiform mit schnell freisetzendem Kern beschrieben oder hergestellt hat. Durch die vorliegende Erfindung kann der Patient in die Lage versetzt werden, das Medikament nur einmal täglich applizieren zu müssen, was insbesondere bei Dauertherapie eine sicherere und angenehmere Behandlungsart darstellt.

Die Kurven der Abb. 1 zeigen für einige ausgewählte erfindungsgemäße Beispiele das Prinzip der diskontinuierlichen Wirkstofffreisetzung, insbesondere die gezielten Freisetzungsschübe, die praktisch unabhängig vom pH-Wert einige Stunden nach der Applikation eingestellt werden können.

Beispiele

Beispiel 1

Kern

| | |
|---|---|
| Nitrendipin mikrofein | 8,0 mg |
| Laktose | 8,0 mg |
| Cellulose mikrokristallin | 8,0 mg |
| quervernetztes PVP | 16,0 mg |

werden gemischt und granuliert mit:

| | |
|---|---|
| PVP 25 | 4,0 mg |
| Natriumlaurylsulfat | 0,8 mg |
| Wasser | q.s. |

nach dem Trocknen wird zugemischt

Magnesiumstearat 0,2 mg

und zu Tabletten verpreßt:

| | |
|---|---|
| Gewicht | 45,0 mg |
| Format ø | 5,0 mm |

Mantel:

| | |
|---|---|
| Hydroxypropylcellulose Typ L | 50,0 mg |
| Hydroxypropylcellulose Typ M | 87,5 mg |
| Laktose | 111,0 mg |

werden gemischt und mit Wasser granuliert (gegebenenfalls kann ein Teil der Hydroxypropylcellulose zur Granulation verwendet werden). Nach dem Trocknen wird gemischt mit:

Magnesiumstearat 1,5 mg

Kern und Mantelgranulat werden auf einer Mantel-Tablettenpresse zu Manteltabletten verpreßt:

Gewicht 295,0 mg
Format ø 9,0 mm

Initialdosis:

Auf die Manteltablette wird 4 mg Nitrendipin auflackiert mittels üblicher Verfahren. Der Lack besteht aus Nitrendipin, Hydroxypropylmethylcellulose und Polyethylenglycol. Er setzt den Wirkstoff schnell frei.

Die in vitro Freisetzung des Wirkstoffes aus dieser lackierten Manteltablette zeigt Abb. 1.

Beispiel 2

Kern:

Nifedipin mikrofein 5,0 mg
Laktose 38,8 mg
Maisstärke 15,0 mg

werden gemischt und granuliert mit Stärkekleister:

Maisstärke 1,0 mg
Wasser q.s.

nach dem Trocknen wird zugemischt:

Magnesiumstearat 0,2 mg
Cellulose mikrokristallin 5,0 mg

und zu Tabletten verpreßt:

Gewicht 65,0 mg
Format ø 6,0 mm

Mantel:

Hydroxypropylcellulose Typ M 126,0 mg
Laktose 145,6 mg

werden gemischt und mit Wasser granuliert (gegenbenenfalls kann ein Teil der Hydroxypropylcellulose zur Granulation verwendet werden). Nach dem Trocknen wird gemischt mit:

Magnesiumstearat 8,4 mg

Kern und Mantelgranulat werden auf einer Mantel-Tablettenpresse zu Manteltabletten verpreßt:

Gewicht 345,0 mg
Format ø 9,0 mm

Initialdosis:

Auf die Manteltabletten wird eine zweite Schicht wirkstoffhaltiges Granulat (Zusammensetzung wie unter "Kern" beschrieben) aufgepreßt, Dosis Nifedipin 5 mg.

Die in vitro Freisetzung des Wirkstoffes aus dieser Zweischichtmanteltablette zeigt Abb. 1.

Beispiel 3

Kern:

| | |
|---|---|
| Nisoldipin mikrofein | 10,0 mg |
| Laktose | 12,0 mg |
| Cellulose mikrokristallin | 10,0 mg |
| PVP quervernetzt | 7,5 mg |

werden gemischt und granuliert mit:

| | |
|---|---|
| PVP 25 | 2,5 mg |
| Natriumlaurylsulfat | 0,5 mg |
| Wasser | q.s. |

nach dem Trocknen wird zugemischt:

| | |
|---|---|
| Magnesiumstearat | 0,2 mg |
| PVP quervernetzt | 2,3 mg |

und zu Tabletten verpreßt:

| | |
|---|---|
| Gewicht | 45,0 mg |
| Format ø | 5 mm |

Mantel:

| | |
|---|---|
| Hydroxypropylcellulose Typ L | 31,0 mg |
| Hydroxipropylcellulose Typ M | 106,0 mg |
| Laktose | 111,5 mg |

werden gemischt und mit Wasser granuliert (gegebenenfalls kann ein Teil der Hydroxy propylcellulose zur Granulation verwendet werden). Nach dem Trocknen wird gemischt mit:

| | |
|---|---|
| Magnesiumstearat | 1,5 mg |

Kern und Mantelgranulat werden auf einer Mantel-Tablettenpresse zu Manteltabletten verpreßt:

| | |
|---|---|
| Gewicht | 295 mg |
| Format ø | 9 mm |

Initialdosis:

Auf die Manteltabletten wird mit den üblichen Methoden und Lackhilfsstoffen (vgl. z.B. Beispiel 1) Nisoldipin 10 mg auflackiert.

Beispiel 4

Kern:

wie Beispiel 3, aber Nitrendipin mikrofein anstelle von Nisoldipin mikrofein enthaltend.

Mantel:

wie Beispiel 3

Manteltablette:

Gewicht: 295,0 mg
Format ø 9,0 mm

Initialdosis:

wie Beispiel 3, aber Nitrendipin mikrofein anstelle von Nisoldipin mikrofein.

Die in vitro Freisetzung des Wirkstoffes aus diesen lackierten Manteltabletten zeigt Abb. 1.

Beispiel 5

Kern:

wie Beispiel 2, aber zusätzlich 2,5 mg 2-Methyl-4-(4-oxo-2-phenyl-4H-thiochromen-8-yl)-5-oxo-1,4,5,7-tetrahydrofuro-[3,4-b]-pyridin-3-carbonsäureethylester enthaltend (Ausgleich zum Kerngewicht von 65 mg mit Maisstärke)

Mantel:

wie Beispiel 2

Initialdosis:

Auf die Manteltabletten wird eine zweite Schicht wirkstoffhaltiges Granulat (Zusammensetzung wie unter "Kern" beschrieben) aufgepreßt.

Beispiel 6

Kern:

wie in Beispiel 4

Mantel:

Methylcellulose 250,0 mg
Laktose 65 mg
werden gemischt und mit Wasser granuliert (gegebenenfalls kann ein Teil der Methylcellulose zur Granulation verwendet werden). Nach dem Trocknen wird gemischt mit:
Magnesiumstearat 5,0 mg

Kern und Mantelgranulat werden auf einer Mantel-Tablettenpresse zu Manteltabletten verpreßt:
Gewicht 365,0 mg
Format ø 9,0 nm

Initialdosis:

wie in Beispiel 4

Beispiel 7

Kern:

wie in Beispiel 5

Mantel:

vorverkleisterte Stärke 225 mg
Laktose 90 mg

werden gemischt und mit Wasser granuliert. Nach dem Trocknen wird gemischt mit:

Magnesiumstearat 5 mg

und zu Manteltabletten verpreßt:

Gewicht 385,0 mg
Format ø 9,0 mm

Beispiel 8

In einem Rotorgranulator werden Pellets mit folgendem Aufbau hergestellt:

Kern:

Durchmesser: 1.00 - 1.25 mm

Zusammensetzung:

Nitrendipin mikrofein 70 %
Hydroxypropylcellulose Typ L 28 %
Natriumlaurylsulfat 2%

Mantel:

Zusammensetzung:

vorverkleisterte Stärke 30 %
Hydroxypropylcellulose Typ M 40 %
hydriertes Rizinusöl 30 %
Auftragsmenge (bezogen auf das Kerngewicht):
je nach gewünschter Verzögerungszeit, beliebig einstellbar, z. B. 300%

Beispiel 9

In einem Dragierkessel werden Pellets mit folgendem Aufbau hergestellt:

Kern:

Durchmesser: 0.63-0.8 mm

Zusammensetzung:

Nisoldipin mikrofein 70 %
Hydroxypropylcellulose Typ L 28 %
Natriumlaurylsulfat 2 %

Mantel:

Zusammensetzung:

hydriertes Rizinusöl 70 %
Hydroxypropylcellulose Typ M 30 %

Auftragsmenge (bezogen auf das Kerngewicht):

1. 0 %
2. 100 %
3. 200 %
4. 300 %

Die Pellets 1-4 werden so gemischt, daß 30 mg Nisoldipin mikrofein sich wie folgt auf die Pellets aufteilen:

| | | | | | |
|---|---|---|---|---|---|
| 13 | % der | Dosis | - | Pellet | 1 |
| 16 | % " | " | | " | 2 |
| 27 | % " | " | | " | 3 |
| 44 | % " | " | | " | 4 |

Das Pelletgemisch wird in Hartgelatinesteckkapseln abgefüllt.

Beispiel 10

Kern:

Nimodipin mikrofein 70 %
Hydroxypropylcellulose Typ L 28 %
Natriumlaurylsulfat 2 %
werden gemischt und durch Pelletisierung mit Wasser als Granulierflüssigkeit in sphärische Partikeln mit einem mittleren Durchmesser von 0,5 - 1,5 mm überführt.

Mantel

In einem kontinuierlich arbeitenden Rotorgranulator werden gleichzeitig Nimodipin-Kerne und Mantelpulver gemischt sowie Wasser als Granulierflüssigkeit zugegeben. Das Mantel-Pulvergemisch ist wie folgt zusammengesetzt:

10a) Cellulose mikrokristallin 86 %
Calciumstearat 9 %
Hydroxypropylcellulose Typ M 5 %

10b) Reisstärke 20 %
Rizinusöl hydriert 50 %
Hydroxypropylcellulose Typ M 30 %

10c) Cellulose mikrokristallin 70 %
Stearinsäure 15 %
Natriumcarboxymethylcellulose Hydroxypropylmethylcellulosegemisch (1:1) 10%
10d) vorverkleisterte Maisstärke 30 %
Hydroxypropylcellulose Typ M 40 %
hydriertes Rizinusöl 30 %
10e) hydriertes Rizinusöl 70 %
Hydroxypropylcellulose Typ M 30 %.

Das Mantelpulvergemisch der Beispiele 10a - e wird in folgenden Mengen auf die Kerne aufgetragen:
Beispiel 10 a - 150 % des Kerngewichts
10 b - 250 % des Kerngewichts
10 c - 100 % des Kerngewichts
10 d - 300 % des Kerngewichts
10 e - 150 % des Kerngewichts.

## Ansprüche

1. Feste Arzneizubereitung mit lang anhaltender Wirkung für 1 bis 2 mal tägliche Applikation enthaltend mindestens ein Dihydropyridin der allgemeinen Formel I.

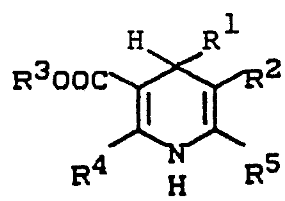

I,

in welcher
$R^1$ für einen Phenylrest steht, der ein oder zwei gleiche oder verschiedene Substituenten aus der Gruppe Nitro, Halogen oder Trifluormethyl trägt oder für

den Rest 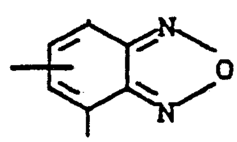 oder

für 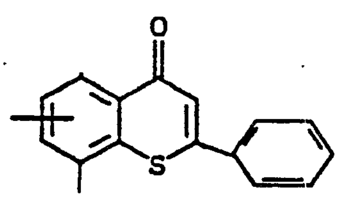 steht,

$R^2$ für eine Nitrogruppe steht oder für den Rest $COOR_6$ steht, wobei
$R_6$ Alkyl mit 1 bis 10 C-Atomen bedeutet, welches gegebenenfalls substituiert ist durch Alkoxy mit 1 bis 4 C-Atomen oder durch ein oder mehrere Halogene
oder wobei
$R^2$ gemeinsam mit $R^5$ für die Lactongruppe $-CO-O-CH_2-$ steht,
$R^3$ für Alkyl mit 1 bis 10 C-Atomen steht, welches gegebenefalls substituiert ist durch Alkoxy mit 1 bis 4 C-Atomen oder durch ein oder mehrere Fluore und
$R^4$ und $R^5$ gleich oder verschieden sind und jeweils für Alkyl mit 1 bis 4 C-Atomen, welches gegebenenfalls durch Hydroxy substituiert ist, stehen,
bestehend aus einem schnell freisetzenden Kern und einem den Freisetzungsbeginn verzögernden Mantel, wobei diese Darreichungsform
a) aus einem Kern besteht, der mindestens eins der oben genannten Dihydropyridine in schnell freisetzender Form enthält
und
b) aus einem um den Kern liegenden Mantel besteht, der keinen Wirkstoff enthält und der sich in wäßrigem Milieu nur langsam auflöst,
und
c) gegebenenfalls auf dem Mantel zusätzlich eine schnell freisetzende Initialdosis eines Dihydropyridinwirkstoffs enthält,
wobei der Durchmesser der Zubereitungsform zwischen 0,5 und 15 mm liegt.

2. Feste Arzneizubereitungen gemäß Anspruch 1, dadurch gekennzeichnet, daß sie im Kern 20 bis 100 Gewichtsprozent des gesamten Wirkstoffgehaltes enthalten.

3. Arzneizubereitung gemäß Anspruch 1 enthaltend insgesamt 1 bis 200 mg Dihydropyridinwirkstoff.

4. Arzneizubereitung gemäß Anspruch 1, dadurch gekennzeichnet, daß der schnell freisetzende Kern den Wirkstoff in amorpher Form oder in fein gemahlener oder mikronisierter kristalliner-Form enthält, gegebenenfalls zusätzlich neben gut wasserlöslichen Hilfsstoffen und/oder Sprengmitteln und/oder Netzmitteln.

5. Arzneizubereitung gemäß Anspruch 1, dadurch gekennzeichnet, daß der schnell freisetzende Kern amorphes Dihydropyridin gemeinsam mit wasserlöslichen Polymeren wie Polyvinylpyrrolidon, Methylcellulose, Hydroxypropylcellulose oder Hydroxypropylmethylcellulose enthält, wobei auf 1 Gew.-Teil Dihydropyridin 2-10 Gew.-Teile der wasserlöslichen Polymere kommen oder wobei der Kern die Dihydropyridinwirkstoffe in kristalliner Form mit einer maximalen mittleren Korngröße von 25 µm enthält.

6. Arzneizubereitung gemäß Anspruch 1, dadurch gekennzeichnet, daß der wirkstofffreie Mantel ein hydrophiles, gelbildendes Polymer und gegebenenfalls weitere übliche Hilfsstoffe enthält.

7. Arzneizubereitung gemäß Anspruch 1, dadurch gekennzeichnet, daß der wirkstofffreie Mantel als hydrophile gelbildende Polymere modifizierte Stärken, celluloseartige Polymere, wie z. B Methylcellulose, Hydroxypropylmethylcellulose, Hydroxypropylcellulose oder Natriumcarboxymethylcellulose, sowie gegebenenfalls als Modulator hydrophobe Hilfsstoffe wie hydriertes Rizinusöl, Calciumstearat, Magnesiumstearat, Stearinsäure, Cetylpalmitat oder Wachse wie Bienenwachs enthält.

8. Verfahren zur Herstellung von festen Arzneizubereitungen gemäß Anspruch 1, dadurch gekennzeichnet, daß man

A) Manteltabletten herstellt, indem man zunächst den wirkstoffhaltigen, schnell freisetzenden Kern mittels üblicher Tablettierprozesse herstellt durch Pressen von Pulvermischungen oder Granulaten und anschließend um diesen Kern mit Hilfe einer Manteltablettenpresse einen wirkstofffreien Placebomantel legt, wobei das Mantelmaterial aus einer rieselfähigen Pulvermischung oder aus einem Granulat besteht und gegebenenfalls auf dieser Manteltablette eine Lackschicht mit üblichen Methoden wie Aufsprühen oder eine weitere Schicht durch Aufpressen einer Pulvermischung oder eines Granulates aufbringt, wobei dieser zusätzliche Schicht gegebenenfalls eine Initialdosis des Wirkstoffs beigemischt ist, oder

B) Mantelpellets herstellt, indem man entweder die Pelletkerne vorher in einem separaten Arbeitsgang hergestellt hat oder direkt in einem Pelletisiergerät oder Granulator kontinuierlich aufbaut und anschließend das Mantelmaterial mittels einer aufbauenden Granulation in einem Granulator oder Pelletisiergerät wie Pelletisierteller, Rotorgranulator, Wirbelschichtgranulator oder Dragierkessel aufbringt.

9. Verfahren gemäß Anspruch 8. B), dadurch gekennzeichnet, daß es in einem Rotorgranulator durchgeführt wird.

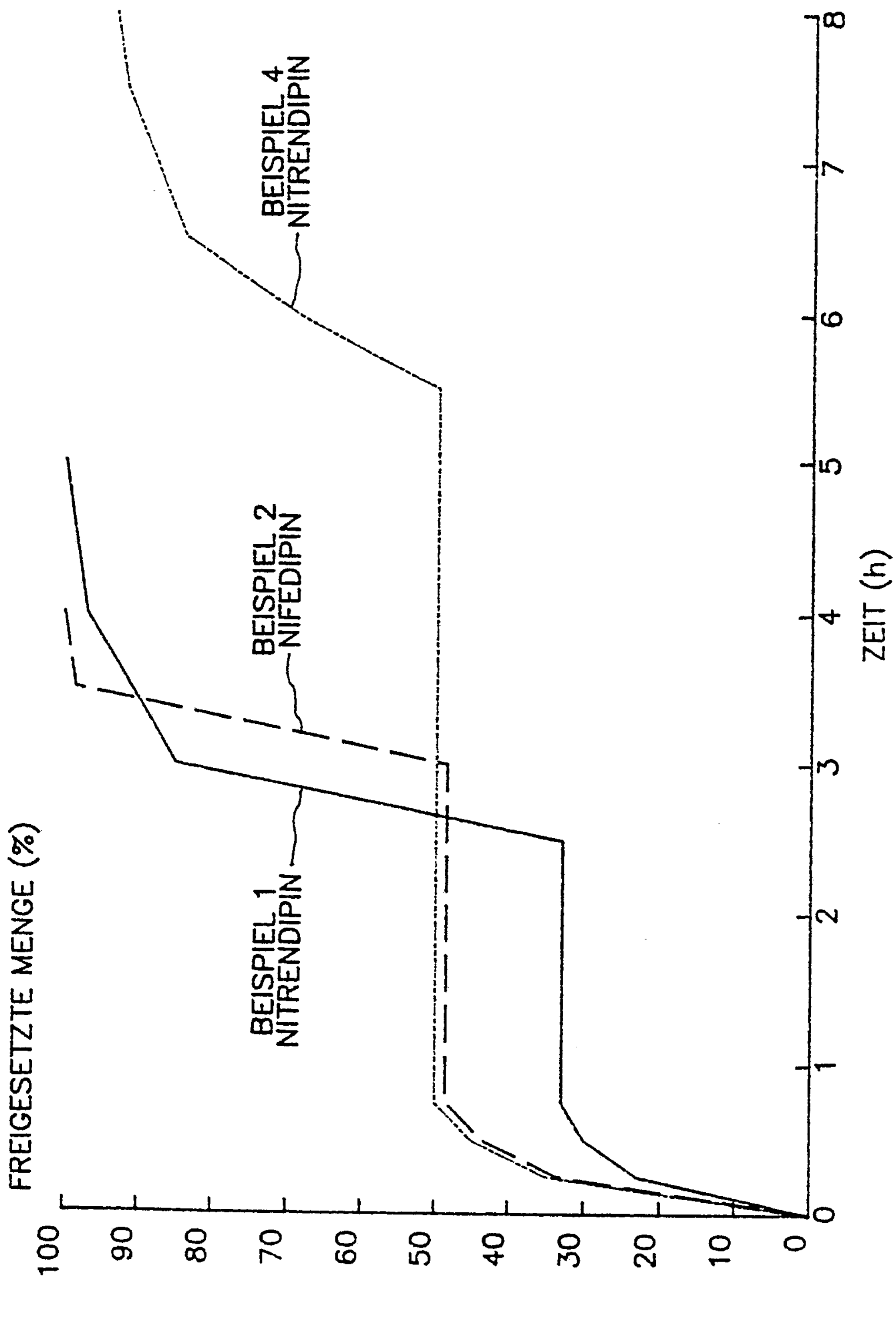
FREIGESETZTE MENGE (%)
100
90
80
70
60
50
40
30
20
10
0
0
1
2
3
4
5
6
7
8
ZEIT (h)
BEISPIEL 1
NITRENDIPIN
BEISPIEL 2
NIFEDIPIN
BEISPIEL 4
NITRENDIPIN

Europäisches Patentamt

# EUROPÄISCHER RECHERCHENBERICHT

Nummer der Anmeldung

EP 88 11 2494

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 205 282 (EUROCELTIQUE S.A.)<br>* Spalte 1, Zeile 46 - Spalte 2, Zeile 26; Spalte 3, Zeilen 29-56; Spalte 5, Zeilen 1-32; Ansprüche 1-16 *<br>--- | 1-9 | A 61 K 31/44<br>A 61 K 9/24<br>A 61 K 9/54 |
| Y | EP-A-0 137 198 (FUJISAWA PHARMACEUTICAL CO. LTD)<br>* Seite 2, Zeile 9 - Seite 3, Zeile 34; Ansprüche 1-5 *<br>--- | 1-9 | |
| Y | EP-A-0 164 588 (BAYER AG)<br>* Seite 3, Zeile 13 - Seite 4, Zeile 7; Seite 6, Zeilen 9-32; Beispiel 4 *<br>--- | 1-9 | |
| Y | EP-A-0 222 411 (TAISHO PHARMACEUTICAL CO. LTD.)<br>* Seite 2, Zeile 27 - Seite 5, Zeile 3 *<br>--- | 1-9 | |
| A | US-A-4 309 405 (PAUL C. GULEY et al.)<br>* Spalte 2, Zeile 22 - Spalte 3, Zeile 51 *<br>----- | | RECHERCHIERTE SACHGEBIETE (Int. Cl.4)<br>A 61 K |

Der vorliegende Recherchenbericht wurde für alle Patentansprüche erstellt

| Recherchenort | Abschlußdatum der Recherche | Prüfer |
|---|---|---|
| DEN HAAG | 29-11-1988 | TZSCHOPPE,D.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)